# EUROPEAN PATENT APPLICATION

(11) **EP 1 504 746 A1**
(43) Date of publication of application: **09.02.2005**
(21) Application number: 03017989.9
(22) Date of filing: 07.08.2003
(51) Int. Cl.: A61K 7/06, A61K 7/50

(54) **Conditioning shampoo composition comprising cetyl PEG/PPG-10/1 dimethicone and a foaming surfactant**

(71) Applicant: KPSS-Kao Professional Salon Services GmbH, 64297 Darmstadt (DE)
(72) Inventor: Molenda, Michael, 60598 Frankfurt (DE); Hoffmann, Martin, 64673 Zwingenberg (DE)

(57) **Abstract**

Aqueous conditioning shampoo composition comprising at least one foaming surfactant selected from anionic, nonionic, and amphoteric or zwitterionic surfactants and cetyl PEG/PPG-10/1 dimethicone as a conditioner is disclosed.

## Description

The present invention concerns a shampoo composition with optimum hair conditioning properties.

Conditioning shampoo compositions have been known for years and have proven very successful on the market.

Such shampoos customarily comprise at least one surface-active substance, in particular an anionic surfactant, and a hair-conditioning polymer, preferably of the cationic type.

Although these products are proven as such, it is still desirable to improve their efficiency, in particular with regard to volume, gloss, combability and body of the hair washed with these products.

Moreover, a shampoo is expected to have good lathering properties and a high lather volume. Finally, shampoos must also be mild, i.e. they must be entirely compatible with the skin and mucous membrane.

It has now been found that a shampoo is obtained which meets all these requirements if this shampoo, in an aqueous carrier, comprises at least one surfactant with a good lathering properties and cetyl dimethicone copolyol (cetyl PEG/PPG-10/1 dimethicone) as a conditioning agent. Surfactants suitable for achieving good lathering properties according to the invention should be chosen from anionic, non-ionic and zwitterionic or amphoteric ones. It is the preferred embodiment of the invention that the main surfactant should be anionic one and the non-ionic and zwitterionic or amphoteric surfactants should be used as cosurfactants for improving foam and/or conditioning properties. Total surfactant concentration in shampoo is in the range of 1 to 50%, preferably 5 to 40% and more preferably 5 to 30% and most preferably 5 to 25% by weight calculated to the total composition. It should be noted that the conditioning shampoo compositions according to the invention can comprise conditioning agents other than cetyl dimethicone copolyol such as cationic surfactants, cationic polymers, silicone oils either non-ionic or cationic, triglycerides. The details of the other conditioning agents can be found below in the description.

Cetyl dimethicone copolyol has been discloses as conditioning agent in emulsion type preparations suitable especially for preparation designed for skin care. EP 836 848 A2 describes cosmetic skin browning and light protection agent comprising cetyl dimethicone copolyol. Furthermore, DE 101 44 235 disclosing water in silicone (W/S) emulsions comprising cetyl dimethicone copolyol. In both documents it is disclosed that those preparations are only suitable for use on skin.

In the prior art so far know, compositions are not disclosed suitable for cleansing and conditioning hair comprising at least one surfactant and cetyl dimethicone copolyol.

Cetyl dimethicone copolyol used in the shampoo compositions of the inventions is known with its trade name Abil^{R} EM 90. It is also know with its INCI name Cetyl PEG/PPG-10/1 dimethicone. Chemically, it is a siloxane modified with polyether and alkyl groups. It has been disclosed to an emulsifier with an HLB value of 5, so that for W/O emulsions or multiple emulsions of W/O/W or O/W/O.

Suitable anionic surfactants within the scope of the invention are contained in an amount ranging from at least 1 to 40%, preferably 5 to 30%, more preferably 5 to 25% by weight calculated to total composition. These surfactants are of the sulfate, sulfonate, carboxylate and alkyl phosphate type, especially, of course, those customarily used in shampoos, for example, the known C₁₀-C₁₈-alkyl sulfates and in particular the respective ether sulfates, for example, C₁₂-C₁₄-alkyl ether sulfate, lauryl ether sulfate, in particular with 1 to 4 ethylene oxide groups in the molecule, furthermore monoglyceride(ether)sulfates, fatty acid amide sulfates, obtained by ethoxylation and subsequent sulfatization of fatty acid alkanolamides, and the alkali salts thereof, as well as salts of long-chain mono-arid dialkyl phosphates, which constitute mild, skin-compatible detergents.

Further anionic surfactants suitable within the scope of the invention are α-olefine sulfonates or the salts thereof, and in particular alkali salts of sulfosuccinic acid semiesters, for example, the disodium salt of monooctyl sulfosuccinate and the alkali salts of long-chain monoalkyl ethoxy sulfosuccinates.

Suitable surfactants of the carboxylate type are alkyl, polyether carboxylic acids and the salts thereof of the formula

R₁ - (C₂H₄O)ₙ - O - CH₂COOX,

wherein R₁ is a C₈-C₂₀-alkyl group, preferably a C₁₂-C₁₄-alkyl group, n is a number from 1 to 20, preferably 2 to 17, and X is H or preferably a cation of the group sodium, potassium, magnesium, ammonium and mono or diethanol amine.

Alkyl amido polyether carboxylic acids of the general formula 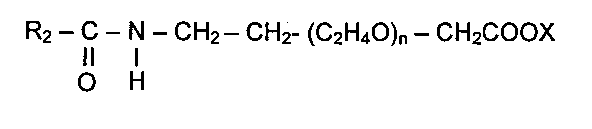 wherein R₂ is a C₈-C₂₀-alkyl group, preferably a C₁₂-C₁₄-alkyl group, n is in particular a number from 1 to 10, preferably 2.5 to 5 and X has the above-named meaning.

Such products have been known and on the market for some time, for example, under the trade name "AKYPO® " and "AKYPO-SOFT®".

C₈-C₂₀-acyl isethionates can also be used alone or in admixture with other surfactants, as well as sulfofatty acids and the esters thereof.

Useful are also mixtures of several anionic surfactants, for example, a mixture of an α- olefine sulfonate and a sulfosuccinate, or an ether sulfate and a polyether carboxylic acid or alkyl amidoether carboxylic acid.

An overview of anionic surfactants used in liquid body and/or hair cleansing products can, moreover, be found in the monography of K.Schrader, "Grundlagen und Rezepturen der Kosmetika", 2nd Ed., (1989, Hüthig Buchverlag), pp. 683 to 691.

The preferred anionic surfactant is ethoxylated fatty alcohol sulfates. The preferred quantities of anionic surfactants in total in the liquid body cleansers according to the invention are in the range of 1 to 40%, in particular of 5 to 30%, especially of 5 to 25% by weight, calculated to the total composition.

In addition to the anionic surfactants, the conditioning shampoo compositions according to the present invention comprise nonionic and amphoteric or zwitterionic surfactants.

Nonionic surfactants suitable are compounds from the category of alkyl polyglucosides with the general formula

R₃-O-(CH₂CH₂O)ₙ-Zₓ,

wherein R₃ is an alkyl group with 8 to 20, preferably 10 to 14 carbon atoms, Zₓ is a saccharide group with 5 to 6 carbon atoms, n stands for a number from 0 to 10, and x is a number between 1 and 5, preferably 1.1 to 2.5.

Further nonionic surfactants useful in the compositions according to invention are C₁₀-C₂₂-fatty alcohol ethoxylates. Especially suited C₁₀-C₂₂-fatty alcohol ethers are the alkyl polyglycol ethers known by the generic terms "Laureth", "Myristeth", "Oleth", "Ceteth", "Deceth", "Steareth" and "Ceteareth" according to the CTFA nomenclature, including addition of the number of ethylene oxide molecules, e.g., "Laureth-16":

The average degree of ethoxylation thereby ranges between about 2.5 and about 25, preferably about 10 and about 20.

Other additionally useful surfactants are, for example, the various sorbitan esters, such as polyethylene glycol sorbitan stearic acid ester, fatty acid polyglycol ester or also mixed condensates of ethylene oxide and propylene oxide, as they are on the market, for example, under the trade name "Pluronics^{R}".

Further useful nonionic surfactants are amineoxides. Such amineoxides are for example C₁₂-C₁₈- alkyl dimethyl amineoxides such as lauryl dimethyl amineoxide, C₁₂-C₁₈- alkyl amidopropyl or ethyl amineoxides, C₁₂-C₁₈ -alkyl di(hydroxyethyl) or (hydroxypropyl) amineoxides, or also amineoxides with ethylene oxide and/or propylene oxide groups in the alkyl chain.

Suitable amineoxides are on the market, for example, under the trade names "Ammonyx® ", "Aromox® " or "Genaminox® ".

Further nonionic surfactant components are fatty acid mono- and dialkanolamides, such as coco fatty acid monoethanolamide and myristic fatty acid monoisopropanolamide.

The preferred nonionic surfactant is of type alkyl polyglucoside. The concentration of nonionic surfactants is typically from 0.5 to 15%, preferably 0.5 - 10%, more preferably 1 - 5% by weight, calculated to the total composition.

In addition to anionic and non-ionic surfactants, conditioning shampoo composition the present invention contains amphoteric or zwitterionic surfactants as cosurfactants in order to improve foam speed and properties such as creaminess. Suitable ones are in particular the various known betaines such as fatty acid amidoalkyl betaines and sulfobetaines; for example lauryl hydroxy sulfobetaine; long-chain alkyl amino acids, such as cocoaminoacetate, cocoaminopropionate and sodium cocoamphopropionate and -acetate have also proven suitable.

In detail it is possible to use betaines of the structure 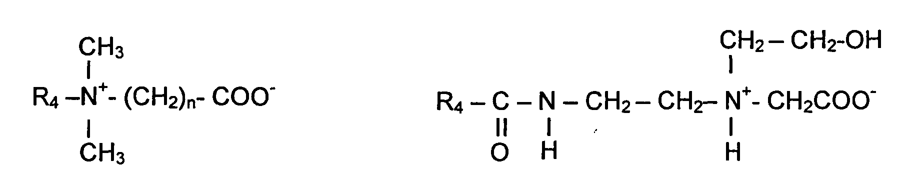 wherein R₄ is a C₈-C₁₈-alkyl group and n is 1 to 3,
sulfobetaines of the structure 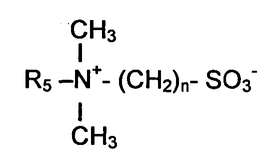 wherein R₅ is a C₈-C₁₈-alkyl group and n is 1 to 3,
and amidoalkyl betaines of the structure 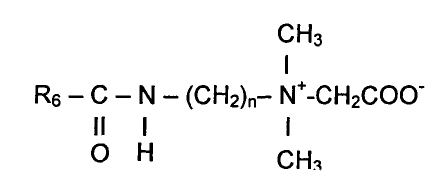 wherein R₆ is a C₈-C₁₈-alkyl group and n is 1 to 3,

Preferred are fatty acid amidoalkyl betaines, in particular cocoamidopropyl betaine, and cocoamphoacetate and -propionate, in particular the sodium salts thereof.

Preferred amphoteric or zwitterionic surfactant is those of betaine derivatives.The concentration of amphoteric or zwitterionic surfactants is from 0.5 to 15%, preferably 0.5 - 10%, more preferably 1 - 8% by weight, calculated to the total composition.

The weight ratio of the main surfactant, being anionic surfactant, to the cosurfactants, being nonionic and amphoteric or zwitterionic surfactants, should be in the range of 10:1 to 1:3, preferably 5:1 to 1:2. Furthermore, the ratio of the anionic surfactant to amphoteric or nonionic surfactants should be in the range of 10:1 to 1:1, preferably 5:1 to 2:1.

Concentration of the conditioning ingredient cetyl dimethicone copolyol, or with INCI name cetyl PEG/PPG-10/1 dimethicone, is in the range of 0.01 - 5%, preferably 0.01 - 4% and more preferably 0.05 - 2.5% by weight, calculated to the total composition.

Conditioning shampoo compositions of the present invention can contain additional cationic polymers as conditioning agents. Suitable polymers are those of cationic polymers best known with their CTFA category name Polyquaternium. Typical examples of those Polyquaternium 6, Polyquaternium 7, Polyquaternium 10, Polyquaternium 11, Polyquaternium 16, Polyquaternium 22 and Polyquaternium 28.

As well those polymers known with their CTFA category name Quaternium are suitable. Those are for example Quaternium-8, Quaternium-14, Quaternium-15, Quatemium-18, Quaternium-22, Quaternium-24, Quaternium-26, Quaternium-27, Quaternium-30, Quaternium-33, Quaternium-53, Quaternium-60, Quaternium-61, Quaternium-72, Quaternium-78, Quaternium-80, Quaternium-81, Quaternium-81, Quaternium-82, Quaternium-83 and Quaternium-84. It has been found out that Quaternium-80 is the best suitable one among the Quaterniums.

It has further been found out that especially those of cationic cellulose type polymers known as Polymer JR type from Amerchol such as Polyquaternium 10 or cationic guar gum known with trade name Jaguar from Rhône-Poulenc and chemically for example Guar hydroxypropyl trimonium chloride, are preferred ones. Furthermore, chitosan and chitin can also be included in the compositions as cationic natural polymers. In this context reference is also made to the cationic polymers disclosed in DE 25 21 960, 28 11 010, 30 44 738 and 32 17 059, as well as to the products described in EP-A 337 354 on pages 3 to 7. It is also possible to use mixtures of various cationic polymers.

The cationic polymers also include the quaternized products of graft polymers from organopolysiloxanes and polyethyl oxazolines described in EP-A 524 612 and EP-A 640 643.

The conditioning shampoo compositions of the present invention can further comprise silicone-conditioning agents. Those can be volatile as well non volatile ones, as well as nonionic and cationic ones are found to be suitable. It should be noted that for the selection compatibility should be evaluated first. Typical examples of those suitable ones are of Dow Corning^{R} series, as well dimethicone, phenyldimethicone, cyclomethicone, polydimethylsiloxane, polydiethylsiloxane, polymethylphenyl siloxane are found to be suitable. As a cationic silicone derivative, amodimethicone is found to be effective.

Conditioning shampoo compositions of the present invention can comprise additionally one or more cationic surfactants as conditioner presented with the general formula 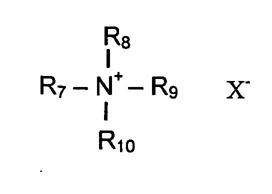 where R₇ is a saturated or unsaturated, branched or non-branched alkyl chain with 8-22 C atoms or

R₁₁ CO NH (CH₂)ₙ

where R₁₁ is saturated or unsaturated, branched or non-branched alkyl chain with 7-21 C atoms and n has value of 1- 4, or

R₁₂CO O (CH₂)ₙ

where R₁₂ is saturated or unsaturated, branched or non-branched alkyl chain with 7-21 C atoms and n has value of 1- 4, and
R₈ is H or unsaturated or saturated, branched or non-branched alkyl chain with 1 - 4 C atoms or

R₁₁ CO NH (CH₂)ₙ

or

R₁₂CO O (CH₂)ₙ

where R₁₁, R₁₂ and n are same as above.
R₉ and R₁₀ are H or lower alkyl chain with 1 to 4 carbon atoms, and X is anion such as chloride, bromide, methosulfate.

Typical examples of those ingredients are cetyl trimethly ammonium chloride, stear trimonium chloride, behentrimoinium chloride, stearamidopropyl trimonuim chloride, dioleoylethyl dimethyl ammonium methosulfate, dioleoylethyl hydroxyethylmonium methosulfate.

The shampoos according to the invention may also comprise additional conditioning substances such as protein hydrolyzates and polypeptides, e.g., keratin hydrolyzates, collagen hydrolyzates of the type "Nutrilan^{R}" or elastin hydrolyzates, as well as also in particular plant protein hydrolyzates, optionally, cationized protein hydrolyzates, e.g., "Gluadin^{R}".

Typical concentration range for any of those additional conditioners of cationic polymers, silicon oil and derivatives and cationic surfactants can be 0.01 - 5% by weight, preferably 0.01 - 3.5% by weight, more preferably 0.05 - 2% and most preferably 0.05 - 1 % by weight calculated to the total composition.

Further conditioning additives are hair conditioning and/or styling polymers. These may be nonionic polymers, preferably alcohol- and/or water-soluble vinyl pyrrolidone polymers, such as a vinyl pyrrolidone homopolymers or copolymers, in particular with vinyl acetate. Useful vinyl pyrrolidone polymers are, e.g., those known by the trade name "Luviskol®", for example, the homopolymers "Luviskol® K 30, K 60 and K 90", as well as the water-or alcohol-soluble copolymers from vinyl pyrrolidone and vinyl acetate, distributed by BASF AG under the trade name "Luviskol® VA 55 respectively VA 64". Further possible nonionic polymers are vinyl pyrrolidone/vinyl acetate/vinyl propionate copolymers such as "Luviskol® VAP 343", vinyl pyrrolidone/(meth)acrylic acid ester copolymers, as well as chitosan derivatives.

As amphoteric polymers are found to be usefull in conditioning shampoo composition of the present invention. They are incorporated alone or in admixture with at least one additional cationic, nonionic or anionic polymer, particularly copolymers of N-octyl acrylamide, (meth)acrylic acid and tert.-butyl aminoethylmethacrylate of the type "Amphomer®"; copolymers from methacryl oylethyl betaine and alkyl methacrylates of the type "Yukaformer®", e.g., the butyl methacrylate copolymer "Yukaformer® Am75"; copolymers from monomers containing carboxyl groups and sulfonic groups, e.g., (meth)acrylic acid and
itaconic acid, with monomers such as mono- or dialkyl amino alkyl(meth)acrylates or mono- or dialkyl aminoalkyl (meth)acrylamides containing basic groups, in particular amino groups; copolymers from N-octyl acrylamide, methyl methacrylate, hydroxypropyl methacrylate, N-tert.-butyl aminoethyl methacrylate and acrylic acid, as well as the copolymers known from US-A 3,927,199, are applicable.

Anionic polymers useful are vinyl alkyl ether, in particular methyl vinyl ether/maleic acid copolmers, obtained by hydrolysis of vinyl ether/maleic acid anhydride copolymers, distributed under the trade name "Gantrez® AN or ES". These polymers may also be partly esterified, for example, "Gantrez® ES 225", the ethyl ester of an ethyl vinyl ether/maleic acid copolymer, or the butyl or isobutyl ester thereof.

Further useful anionic polymers are in particular vinyl acetate/crotonic acid or vinyl acetate/vinyl neodecanoate/crotonic acid copolymers of the type "Resyn®"; sodium acrylate/vinyl alcohol copolymers of the type "Hydagen® F", sodium polystyrene sulfonate, e.g., "Flexan® 130"; ethyl acrylate/acrylic acid/N-tert.-butyl acrylamide copolymers of the type "Ultrahold®"; vinyl pyrrolidone/vinyl acetate/itaconic acid copolymers, acrylic acid/aryl amide copolymers or the sodium salts thereof of the type "Reten®"; etc.

Conditioning shampoo composition of the present invention can be transparent as well a pearly. Transparency of the composition is judged by naked eye in a transparent shampoo bottle with a thickness not more than 5 cm. In the case a transparent appearance is wished, the following ingredients are not essential. However, pearl-shiny appearance is achieved with those dispersed in shampoo compositions in crystalline form, i.e. so called pearl-shine agents. The preferred once are PEG-3 distearate and ethylene glycol distearate. The concentration of those can typically be from 0.1 to 3%, preferably 0.5 to 2% by weight, calculated to the total composition.

The pH of shampoo compositions according to the invention is in the range of 2 to 8, preferably 2 to 6, more preferably 3 to 6. For adjusting the pH of the said conditioning shampoo composition, following ingredients can be used: Organic acids such as citric acid, lactic acid, tartaric acid, malic acid, maleic acid, fumaric acid, levulinic acid, butyric acid and hydroxy butyric acids, valeric acid, oxalic acid, succinic acid, mandelic acid, glycolic acid, glucuronic acid, propionic acid, salicylic acid or acetic acid or inorganic acids such as hydrochloric acid, phosphoric acid, sulphuric acid, nitric acid. Concentration of the organic and/or inorganic acids or their mixtures should be chosen in a way that shampoo composition reaches the desired pH value as given above. Typically concentration for acids can be 0.01 - 3% by weight, preferably 0.05 - 2% by weight, more preferably 0.05 - 1 % by weight calculated to the total composition. The pH of the shampoo composition can also be adjusted to the required pH by using alkaline solution such as sodium hydroxide, potassium hydroxide or their salts with those acids mentioned above in the case that at the selected acid concentration pH of the composition is lower than that of the aimed value. In the case that salts are used for adjusting pH, concentration of salts should not exceed 3% by weight.

The shampoo compositions may contain organic solvents such as ethanol. propanol, isopropanol, benzyl alcohol, benzyloxyethanol, ethoxydiglycol, alkylene carbonates such as ethylene carbonate and propylene carbonate, phenoxyethanol, butanol, isobutanol, cyclohexane, cyclohexanol, hexyleneglycol, ethylenecarbonate, ethyleneglycol monoethylether, ethylene glycol monobutyl ether, ethylene glycol monophenyl ether, 1-phenylethylalcohol, 2-phenylethylalcohol, o-methoxyphenol. Concentration of organic solvents in the shampoo composition should not exceed 5% by weight.

Solubilizers may be added to the compositions especially when oily substances are chosen as conditioning agents and fragrance oils with highly lipophilic properties. Typical solubilizers may be hydrogenated castor oil known with the trade mark Cremophor RH series from BASF. It should be noted that as well the surfactant mixture can be a good solubilizer for fragrance oils. Typical concentration of the solubilizers can be in the range of 0.01 - 2% by weight, preferably 0.1 - 1% by weight, in the case additional solubilizer used as mentioned above other surfactants used for shampooing purposes.

The conditioning shampoo composition may contain active ingredients selected from moisturisers, sequestering agents, antidandruff agents, and natural ingredients showing conditioning benefits.

The moisturising agents are selected from panthenol, polyols, such as glycerol, polyethylene glycols with molecular weight 200 to 20,000. The moisturising ingredients can be included in the conditioner compositions at a concentration range of 0.01 - 2.5% by weight calculated to the total composition.

The sequestering agents are selected from polycarboxy acids. The preferred one is ethylene diamine tetraacetic acid, EDTA. Typical useful concentration range for sequestering agents is of 0.01 - 2.5% by weight calculated to the total composition.

Antidandruff agent is preferably piroctone olamine (Octopirox). Other antidandruff agents such as climbazol can also be used as long as those are soluble in the conditioner compositions or can be solubilized with the aid of the solublizers mentioned above. Typical useful concentration range for sequestering agents is between 0.2 - 1% by weight calculated to the total composition.

The viscosity of the conditioning shampoo compositions according to the invention is in the range of 1,000 and about 20,000 mPa.s at 20°C, preferably 1,000 to 10,000, in particular 1,500 to 7,500 mPa.s at 20°C, and most preferred 2,000 to 5,000 measured with Brookfield or Höppler viscosimeters at a shear rate of 10 sec⁻¹.

It is self-understood that the shampoos according to the invention may comprise all substances customarily used in such compositions.

Examples of such substances are complex formers, dyestuffs for coloring shampoo mainly of anionic character, preservatives, viscosity regulants (thickening agents), such as inorganic salts, to the extent they are not already contained in the original surfactant mixtures, fragrances, moisture retainers, plant and animal oils, such as jojoba oil, etc. A list of such additives can also be found in Schrader, I.c., on pp. 695 to 722.

The following Examples illustrate the invention. The products according to the invention are prepared by mixing the individual components in water, whereby it is also possible to use pre-mixtures of various ingredients.

### Example 1

| | |
|---|---|
| C₁₂-C₁₄-Fatty alcohol ether sulfate | 10.0 (% by wt.) |
| C₈-C₂₂-Alkyl glucoside (P.D.:~ 1.5) | 5.0 |
| Sodium lauroyl glutamate | 2.0 |
| Cocoamidopropyl betaine | 3.2 |
| Cetyl PEG/PPG-10/1 dimethicone | 0.5 |
| Cationic polymer (Polyquaternium-7) | 0.1 |
| Sodium benzoate | 0.6 |
| Sodium sorbate | 0.3 |
| Peach oil | 0.1 |
| Perfume | 0.7 |
| PEG-60-hydrogenated castor oil | 0.5 |
| Benzophenone-3 | 0.1 |
| Water | ad100.0 |

This shampoo was compared in a known half-head double blind test on 10 test persons with a shampoo containing no cetyl PEG/PPG-10/1 dimethicone, but a silicon oil with a trade name Silicon oil AK500 from Wacker Chemie, and with a CTFA name dimethicone, is added at the same concentration.

The separate evaluation by two hairdressers resulted in a preference of 9:1 in favor of the shampoo according to the invention with regard to wet and dry combability, smoothness and elasticity of the wet, washed hair, and of 8:2 in favor of the shampoo according to the invention with regard to volume, bounce, shine and smoothness and softness of the dried hair.

### Example 2

| | |
|---|---|
| Sodium lauryl ether sulfate | 10.0 (% by wt.) |
| Sodium lauroyl glutamate | 4.0 |
| C₁₂-C₁₄-alkyl glucoside (P.D.:~ 1.4) | 3.0 |
| Cocoamidopropyl betaine | 3.5 |
| Cetyl PEG/PPG-10/1 dimethicone | 0.7 |
| Cationic polymer (Polyquaternium-11 ) | 0.2 |
| PEG-3 distearate | 0.8 |
| Perfume | 0.6 |
| Sodium benzoate | 0.6 |
| Citric acid | 0.6 |
| Water | ad 100.0 |

A shampoo with very good lathering capability and hair conditioning properties was obtained.

### Example 3

| | |
|---|---|
| Sodium lauryl ether carboxylate (10EO) | 5.0 (% by wt.) |
| Disodium lauryl ether sulfosuccinate | 3.5 |
| Decylglucoside (P.D.:~ 1.4) | 2.5 |
| Sodium lauroyl glutamate | 3.0 |
| Cocoamidopropyl betaine | 3.0 |
| Cetyl PEG/PPG-10/1 dimethicone | 0.5 |
| Cationic polymer (Polyquaternium-1 0) | 0.2 |
| Sodium benzoate | 0.6 |
| Citric acid | 0.5 |
| Perfume | 0.5 |
| Water | ad 100.0 |

The properties of this shampoo corresponded with those according to Examples 1 and 2.

### Example 4

| | |
|---|---|
| Sodium lauryl ether sulfate | 10.5 (% by wt.) |
| Sodium lauroyl sarcosinate | 4.0 |
| Sodium lauroyl glutamate | 1.0 |
| C₁₂-C₁₄-alkyl glucoside (P.D.:~1.5) | 2.5 |
| Cocodimethyl amineoxide | 1.5 |
| Sodium cocoamidopropylbetaine | 2.5 |
| Cetyl PEG/PPG-10/1 dimethicone | 0.5 |
| Cetrimonium chloride | 0.5 |
| Preservative | 0.5 |
| Citric acid | 0.5 |
| Lactic acid | 0.1 |
| Pyrrolidone carboxylic acid | 0.1 |
| Glycolic acid | 0.1 |
| Malic acid | 0.1 |
| Perfume | 0.5 |
| Water | ad 100.0 |

This shampoo provides the hair with gloss, volume and excellent wet and dry combability as well good styling properties are observed.

### Example 5

| | |
|---|---|
| Sodium lauryl ether sulfate | 10.0 (% by wt.) |
| Sodium lauroyl glutamate | 4.0 |
| C₁₂-C₁₄-alkyl glucoside (P.D.:~ 1.4) | 3.0 |
| Cocoamidopropyl betaine | 3.5 |
| Cetyl PEG/PPG-10/1 dimethicone | 0.7 |
| Dimethicone | 0.5 |
| PEG-3 distearate | 0.8 |
| Perfume | 0.6 |
| PEG 160 hydrogenated castor oil | 0.6 |
| Sodium benzoate | 0.6 |
| Citric acid | 0.6 |
| Water | ad 100.0 |

This shampoo provides the hair with gloss, volume and excellent wet and dry combability.

### Example 6

| | |
|---|---|
| Sodium lauryl ether sulfate | 10.0 (% by wt.) |
| Sodium lauroyl glutamate | 4.0 |
| C₁₂-C₁₄-alkyl glucoside (P.D.:~ 1.4) | 3.0 |
| Cocoamidopropyl betaine | 3.5 |
| Cetyl PEG/PPG-10/1 dimethicone | 0.7 |
| Dimethicone | 0.5 |
| Panthenol | 1.0 |
| PEG-3 distearate | 0.8 |
| Perfume | 0.6 |
| PEG 160 hydrogenated castor oil | 0.6 |
| Sodium benzoate | 0.6 |
| Citric acid | 0.6 |
| Water | ad 100.0 |

This shampoo is found to be suitable especially for dry hair and provides the hair with gloss, volume and excellent wet and dry combability. Hair feels excellently soft after drying.

## Claims

1. Aqueous conditioning shampoo composition for hair **characterized in that**, that it comprises at least one foaming surfactant and cetyl-PEG/PPG-10/1 dimethicone.

2. Aqueous conditioning shampoo composition according to claim 1 **characterized in that** that foaming surfactants are selected form anionic, nonionic and amphoiteric and zwitterionic ones.

3. Aqueous conditioning shampoo composition according to claims 1 and 2 **characterized in that** concentration of foaming surfactants is in the range of 1 to 50% by weight calculated to the total composition.

4. Aqueous conditioning shampoo composition according to any of the preceding claims **characterised in that** concentration of cetyl PEG/PPG-10/1 dimethicone is in the range of 0.01 to 5% by weight calculated to the total composition.

5. Aqueous conditioning shampoo composition according to any of the preceding claims **characterized in that** the ratio between the anionic main surfactant and the nonionic and amphoteric or zwitterionic cosurfactants is in the range of 10:1 to 1:3.

6. Aqueous conditioning shampoo composition according to any of the preceding claims **characterized in that** the ratio between the anionic main surfactant and the nonionic or amphoteric or zwitterionic cosurfactant is in the range of 10:1 to 1:1.

7. Aqueous conditioning shampoo composition according to any of the preceding claims **characterized in that** it is a transparent composition.

8. Aqueous conditioning shampoo composition according to any of the preceding claims **characterized in that** it is a non-transparent pearly composition and contains perlizing agents at a concentrion of 0.1 to 2% by weight caluculated to total composition.

9. Aqueous conditioning shampoo composition according to any of the preceding claims **characterized in that** it has a pH in the range of 2 to 8.

10. Aqueous conditioning shampoo composition according to any of the preceding claims **characterized in that** it comprises additional conditioning agents selected from cationic polymers, cationic surfactants and silicone oils or siloxanes.

11. Aqueous conditioning shampoo composition according to any of the preceding claims **characterized in that** it has a viscosity of 1,000 to 20,000 mPa.s at 20°C measured with Brookfield or Höppler viscosimeters at a shear rate of 10 sec⁻¹.

12. Aqueous conditioning shampoo composition according to any of the preceding claims **characterized in that** it contains as a main anionic surfactant ethoxylated fatty alcohol sulfate.

13. Aqueous conditioning shampoo composition according to any of the preceding claims **characterized in that** it contains as a nonionic cosurfactant alkyl polyglucoside

14. Aqueous conditioning shampoo composition according to any of the preceding claims **characterized in that** it contains as an amphoteric or zwitterionic surfactant betaine derivatives.

15. Aqueous conditioning shampoo composition according to any of the preceding claims **characterized in that** it contains dyestuffs for coloring shampoo mainly of anionic character, preservatives, viscosity regulants (thickening agents), such as inorganic salts.

16. Aqueous conditioning shampoo composition according to any of the preceding claims **characterized in that** it contains organic solvents at a concentration not exceeding 5% by weight calculated to the total composition.

17. Aqueous conditioning shampoo composition according to any of the preceding claims **characterized in that** it contains active ingredients selected from moisturisers, sequestering agents, antidandruff agents, and natural ingredients showing conditioning benefits.
